# EUROPÄISCHE PATENTSCHRIFT

(11) **EP 1 472 343 B1**
(45) Veröffentlichungstag und Bekanntmachung des Hinweises auf die Patenterteilung: **11.04.2007**
(21) Anmeldenummer: 03704520.0
(22) Anmeldetag: 03.02.2003
(51) Int. Cl.: C12N 5/06

(54) **VERFAHREN ZUR HERSTELLUNG EINES NERVENTRANSPLANTATS**
METHOD FOR THE PRODUCTION OF A NERVE TRANSPLANT
PROCEDE DE PRODUCTION D'UNE GREFFE NERVEUSE

(30) Priorität: 06.02.2002 DE 10204966
(43) Veröffentlichungstag der Anmeldung: 03.11.2004
(73) Patentinhaber: Schneider, Wolfgang, 32049 Herford (DE); Fansa, Hisham, 33602 Bielefeld (DE)
(72) Erfinder: Wolfgang Schneider, 32049 Herford (DE)
(74) Vertreter: Elbertzhagen, Otto
(86) Internationale Anmeldenummer: PCT/EP2003/001041
(87) Internationale Veröffentlichungsnummer: WO 2003/066843

(56) Entgegenhaltungen:
- US-A- 5 721 139
- FANSA HISHAM ET AL: "Influence of insulin-like growth factor-I (IGF-I) on nerve autografts and tissue-engineered nerve grafts" MUSCLE AND NERVE, Bd. 26, Nr. 1, Juli 2002 (2002-07), Seiten 87-93, XP009024018 ISSN: 0148-639X
- FANSA H ET AL: "Acellular muscle with Schwann-cell implantation: an alternative biologic nerve conduit." JOURNAL OF RECONSTRUCTIVE MICROSURGERY. UNITED STATES OCT 1999, Bd. 15, Nr. 7, Oktober 1999 (1999-10), Seiten 531-537, XP009024006 ISSN: 0743-684X
- FANSA H ET AL: "Successful implantation of Schwann cells in acellular muscles." JOURNAL OF RECONSTRUCTIVE MICROSURGERY. UNITED STATES JAN 1999, Bd. 15, Nr. 1, Januar 1999 (1999-01), Seiten 61-65, XP009024238 ISSN: 0743-684X
- CALDER J S ET AL: "Nerve-muscle sandwich grafts: the importance of Schwann cells in peripheral nerve regeneration through muscle basal lamina conduits." JOURNAL OF HAND SURGERY (EDINBURGH, LOTHIAN) SCOTLAND AUG 1995, Bd. 20, Nr. 4, August 1995 (1995-08), Seiten 423-428, XP009024240 ISSN: 0266-7681
- FENELEY M R ET AL: "THE ROLE OF SCHWANN CELLS IN THE REGENERATION OF PERIPHERAL NERVE AXONS THROUGH BASAL LAMINA GRAFTS" EXPERIMENTAL NEUROLOGY, Bd. 114, Nr. 3, 1991, Seiten 275-285, XP009024012 ISSN: 0014-4886

## Beschreibung

### TECHNISCHES GEBIET

Die Erfindung betrifft ein Verfahren zur Herstellung eines Nerventransplantats, bei dem die Basalmembranen von Muskeln mit Schwannschen Zellen besiedelt und die besiedelte Basalmembranstruktur in eine Nährlösung eingelegt wird.

### STAND DER TECHNIK

In der Literatur (Journal of Reconstructive Microsurgery, Bd. 15, Nr. 7, 1999, S. 531-537) ist es bekannt, daß man zur Herstellung von Nerventransplantaten Basalmembranen mit Schwannschen Zellen besiedeln kann. Ein praktizierbares Verfahren zur Besiedelung ist bisher nicht bekannt.

### DIE ERFINDUNG

Der Erfindung liegt die Aufgabe zugrunde, ein Verfahren der als bekannt vorausgesetzten Art zur Herstellung eines Nerventransplantats so auszubilden, daß eine gleichmäßige und einwandfreie Besiedelung der Basalmembranstruktur ermöglicht wird und das fertiggestellte Nerventransplantat gleichmäßig und kontinuierlich in Längsrichtung der Fasern ausgerichtete Schwannsche Zellen aufweist.

Die Lösung dieser Aufgabe erfolgt bei dem Verfahren der als bekannt vorausgesetzten Art dadurch, daß die Schwannschen Zellen kontinuierlich in Längsrichtung der Fasern der Basalmembran über die gesamte Länge des Implantats eingebracht werden und die so besiedelte Basalmembran im Nährmedium verbleibt, bis die Schwannschen Zellen sich gleichmäßig und kontinuierlich in Längsrichtung der Fasern ausgerichtet haben. Das Einbringen der Schwannschen Zellen erfolgt dabei mit Hilfe einer Micro-Injektionsnadel, die in die Basalmembran eingeschoben wird. Während des Injizierens der Schwannschen Zellen werden Basalmembran und Injektionsnadel relativ zueinander bewegt.

Bevorzugte Ausgestaltungen des erfindungsgemäßen Verfahrens werden in den Unteransprüchen beschrieben.

### KURZBESCHREIBUNG DER ZEICHNUNGSABBILDUNG

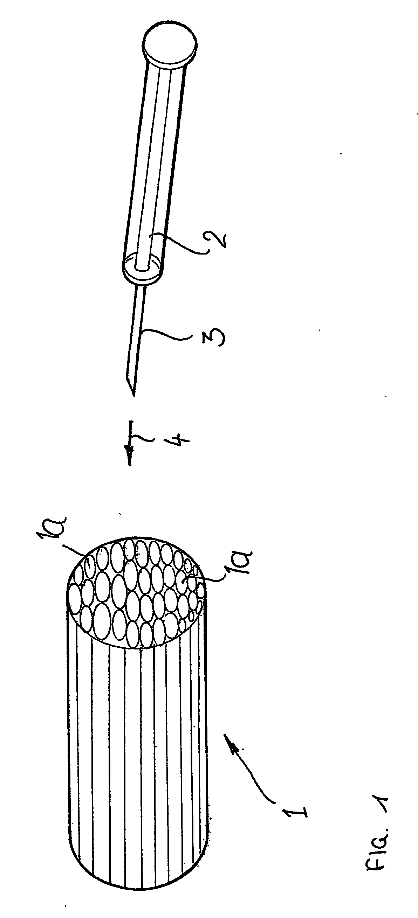

Anhand der einzigen Zeichnung, die lediglich schematisch ist, wird nachstehend eine bevorzugte Ausgestaltung des erfindungsgemäßen Verfahrens beschrieben.

### BESTER WEG ZUR AUSFÜHRUNG DER ERFINDUNG

In der Zeichnung ist mit 1 eine Basalmembran eines Muskels bezeichnet. Diese Basalmembran 1 weist eine Vielzahl von in Längsrichtung der Basalmembran und parallel zueinander verlaufende Fasern 1a auf. Eine Micro-Injektionsnadel 3, die in der Zeichnung verkürzt dargestellt ist, ist am vorderen Ende einer nur schematisch angedeuteten Spritze angesetzt. Die Spritze ist mit Schwannschen Zellen 2 befüllt, die in Richtung des Pfeils 4 aus der Micro-Injektionsnadel 3 herausgedrückt werden können.

Zum Besiedeln der Basalmembran 1 wird die Micro-Injektionsnadel 3 mit den darin enthaltenen Schwannschen Zellen 2 in die Basalmembran 1 eingeführt und bis an das gemäß Zeichnung linke Ende der Basalmembran vorgeschoben. Anschließend wird die Spritze mit der Micro-Injektionsnadel 3 nach rechts zurückgezogen und bei der Rückzugsbewegung werden die Schwannschen Zellen 2 vorsichtig in die Basalmembran 1 injiziert. Dieses Verfahren wird an einer Basalmembran so lange wiederholt, bis die Basalmembran mit Schwannschen Zellen 2 gefüllt ist.

Die so befüllte oder besiedelte Basalmembran 1 wird anschließend in ein Nährmedium eingelegt und verbleibt dort ca. 72 Stunden. In dieser Zeit richten sich die Schwannschen Zellen 2 gleichmäßig und kontinuierlich in Längsrichtun der Fasern 1a der Basalmembran 1 aus.

Beim Einbringen der Schwannschen Zellen 2 ist eine Relativbewegung zwischen Basalmembran 1 und Micro-Injektionsnadel 3 in Längsrichtung dieser Teile erforderlich. Die Relativbewegung erfolgt dabei in Gegenrichtung zur Austrittsrichtung der Schwannschen Zellen 2 aus der Micro-Injektionsnadel 3.

Das Verfahren zum Einbringen wird unter Kontrolle durch ein Mikroskop ausgeführt.

## Patentansprüche

1. Verfahren zur Herstellung eines Nerventransplantats, bei dem die Basalmembranen (1) von Muskeln mit Schwannschen Zellen (2) besiedelt und die besiedelte Basalmembranstruktur in eine Nährlösung eingelegt wird und die Schwannschen Zellen (2) in Längsrichtung der Fasern (1a) der Basalmembran über die gesamte Länge des Implantats eingebracht werden und die so besiedelte Basalmembran (1) im Nährmedium verbleibt, bis die Schwannschen Zellen (2) sich gleichmäßig und kontinuierlich in Längsrichtung der Fasern (1a) ausgerichtet haben,
**dadurch gekennzeichnet,**
**daß** das Einbringen der Schwannschen Zellen (2) kontinuierlich mittels einer Micro-Injektionsnadel (3) in das Zentrum der Basalmembranstrüktur erfolgt, wobei die Micro-Injektionsnadel (3) bis zum Ende der Basalmembran (1) vorgeschoben wird, und daß die Basalmembran (1) und die Injektionsnadel (3) während des Eintragsvorgangs in Längsrichtung der Basalmembran (1) relativ zueinander bewegt werden.

2. Verfahren nach Anspruch 1,
**dadurch gekennzeichnet,**
**daß** die besiedelte Basalmembran (1) ca. 72 Stunden in der Nährlösung verbleibt.

3. Verfahren nach Anspruch 1 oder 2,
**dadurch gekennzeichnet,**
**daß** die Relativbewegung in Gegenrichtung zur Austrittsrichtung (4) der Schwannschen Zellen (2) aus der.Micro-Injektionsnadel (3) erfolgt.

4. Verfahren nach einem oder mehreren der Ansprüche 1 bis 3,
**dadurch gekennzeichnet,**
**daß** das Einbringen der Schwannschen Zellen (2) unter Kontrolle durch ein Mikroskop erfolgt.

5. Verfahren nach einem oder mehreren der Ansprüche 1 bis 4,
**dadurch gekennzeichnet,**
**daß** das Einbringen der Schwannschen Zellen (2) gleichmä-ßig über den gesamten Querschnitt der Basalmembran (1) erfolgt.

## Claims

1. Method for the production of a nerve transplant in which the basal membranes (1) of muscles are colonised by Schwann cells (2) and the colonised basal membrane structure is placed in a nutrient solution and the Schwann cells (2) are applied in the longitudinal direction of the fibres (1a) of the basal membrane over the entire length of the transplant and the basal membrane (1) colonised in this way remains in the nutrient medium until the Schwann cells (2) are aligned homogeneously and continuously in the longitudinal direction of the fibres (1a),
**characterised in that**
introducing the Schwann cells (2) is carried out continuously by means of a micro injection needle (3) into the centre of the basal membrane structure, whereby the micro injection needle (3) is pushed forwards up to the end of the basal membrane (1), and that the basal membrane (1) and the injection needle (3) are moved relative to one another in the longitudinal direction of the basal membrane (1) during the entry procedure.

2. Method according to claim 1,
**characterised in that**
the colonised basal membrane (1) remains in the nutrient solution for about 72 hours.

3. Method according to claim 1 or 2,
**characterised in that**
the relative movement is carried out in the opposite direction to the ejection direction (4) of the Schwann cells (2) from the micro injection needle (3).

4. Method according to one or more of claims 1 to 3,
**characterised in that**
the introduction of the Schwann cells (2) is carried out under supervision through a microscope.

5. Method according to one or more of claims 1 to 4,
**characterised in that**
the introduction of the Schwann cells (2) is carried out homogeneously over the entire cross sectional area of the basal membrane (1).

## Revendications

1. Procédé pour la production d'une greffe nerveuse dans le cadre duquel les membranes basales (1) de muscles sont colonisées avec des cellules de Schwann (2) et la structure de membranes basales, colonisées est mise dans une solution nutritive, et les cellules de Schwann (2) sont amenées, dans la direction longitudinale des fibres (1a) de la membrane basale, sur toute la longueur de la greffe, et la membrane basale (1) ainsi colonisée demeure dans le milieu nutritif jusqu'à ce que les cellules de Schwann (2) soient orientées uniformément et en continu dans la direction longitudinale des fibres (1a)
**caractérisée en ce que**
l'apport des cellules de Schwann (2) s'effectue de manière continue au moyen d'une aiguille de micro-injection (3) dans le centre de la structure de membrane basale, l'aiguille de micro-injection (3) étant poussée en avant jusqu'à l'extrémité de la membrane basale (1), et ladite membrane basale (1) et l'aiguille de micro-injection (3) étant mues l'une par rapport à l'autre, pendant le processus d'introduction, dans la direction longitudinale de la membrane basale (1).

2. Procédé selon la revendication 1,
**caractérisée en ce que**
la membrane basale (1) remplie demeure environ 72 heures dans une solution nutritive.

3. Procédé selon revendication 1 ou 2,
**caractérisée en ce que**
le mouvement relatif s'effectue dans la direction opposée à la direction de sortie des cellules de Schwann (2) de l'aiguille de micro-injection (3).

4. Procédé selon l'une ou plusieurs des revendications 1 à 3,
**caractérisée en ce que**
l'apport des cellules de Schwann (2) s'effectue sous contrôle au moyen d'un microscope.

5. Procédé selon l'une ou plusieurs des revendications 1 à 4,
**caractérisé en ce que**
l'apport des cellules de Schwann (2) s'effectue uniformément, sur toute la section transversale de la membrane basale (1).
